# FASCICULE DE BREVET EUROPEEN

(11) **EP 2 037 847 B1**
(45) Date de publication et mention de la délivrance du brevet: **17.10.2012**
(21) Numéro de dépôt: 07803916.1
(22) Date de dépôt: 21.06.2007
(51) Int. Cl.: A61F 2/66

(54) **PIED PROTHETIQUE**
FUSSPROTHESE
PROSTHETIC FOOT

(30) Priorité: 30.06.2006 FR 0652727
(43) Date de publication de la demande: 25.03.2009
(73) Titulaire: Tourneux, Samuel, 30340 Saint Julien Les Rosiers (FR)
(72) Inventeur: Tourneux, Samuel, 30340 Saint Julien Les Rosiers (FR)
(74) Mandataire: Rhein, Alain
(86) Numéro de dépôt international: PCT/FR2007/051491
(87) Numéro de publication internationale: WO 2008/000997

(56) Documents cités:
- EP-A- 1 149 568
- WO-A2-2006/000211
- FR-A- 2 839 443
- JP-A- 11 277 477
- US-A1- 2005 187 640
- US-B1- 6 511 514
- US-B1- 6 767 370
- US-B1- 6 852 132

## Description

La présente invention concerne le domaine des organes prothétiques, et plus particulièrement celui des pieds prothétiques.

On connaît actuellement des pieds prothétiques destinés au remplacement des pieds naturels. Ces pieds prothétiques sont essentiellement de deux types, les pieds prothétiques rigides munis éventuellement d'une articulation faisant fonction de cheville, et les pieds prothétiques dynamiques.

Les pieds prothétiques rigides sont bien évidemment peu performants et peu confortables d'utilisation, car ils ne présentent de loin pas la souplesse du pied naturel. Pour compenser cela ils sont souvent associés à des butées en matériau élastique, mais ces butées servent essentiellement d'amortisseur, et ne permettent pas une restitution d'énergie en sorte que la pratique de la marche est peu dynamique, et très fatigante.

Les pieds prothétiques dynamiques consistent en des pieds prothétiques monobloc déformables. Ils sont réalisés dans un matériau déformable élastiquement, tel qu'un matériau composite de type fibre de carbone ou fibre de verre. Ils se présentent généralement sous la forme d'au moins une lame ressort fixée rigidement au tibia ou à la cheville, et qui s'étend vers l'avant et/ou vers l'arrière. Ces pieds prothétiques dynamiques permettent une restitution quasi totale de l'énergie utilisée pour les déformer.

Si ces pieds prothétiques dynamiques présentent des avantages par rapport aux pieds prothétiques rigides, ils présentent encore des inconvénients, notamment du fait qu'ils confèrent un déroulé du pas assez éloigné du déroulé naturel, notamment à l'attaque du talon. En effet, selon la conception de ces pieds prothétiques, soit le talon est rigide et, lors notamment des descentes, le porteur reste en appui sur le talon, la partie venant au contact du sol avec un certain retard dû au temps nécessaire pour effectuer la bascule, ce qui engendre un défaut d'appui et une instabilité qui peut être préjudiciable ; soit le talon est souple, souplesse obtenue au travers d'un vérin, ou d'une lame ressort supplémentaire, ce qui permet d'augmenter la stabilité, mais qui par contre provoque un certain déhanchement, du fait de l'écrasement vertical du talon.

On connaît ainsi par le document US 6 767 370 un pied prothétique qui apporte une solution à ces divers inconvénients. Ce pied prothétique comporte d'une part un talon en forme de C et qui présente une certaine élasticité, et d'autre part une partie d'appui au sol, constituée de deux lames ressort montées en parallèle soidarisées par au moins une de leurs extrémités, et entretoisées en zone médiane par un élément mobile longitudinalement, ou de résistance à la compression variable, en sorte d'agir sur les caractéristiques dynamiques de ladite partie d'appui, le déplacement dudit élément mobile ou la modification de sa résistance à la compression étant commandé par l'écrasement dudit talon, dont les effet sont transmis audit élément. L'élément peut être une entretoise apte à être déplacée par une tringlerie, ou bien un élément gonflable sous l'action d'une pompe disposée dans le talon.

Un tel pied prothétique présente des avantages par rapport à ceux connus par ailleurs, en ce sens qu'il permet une modification de la rigidité de la partie d'appui au sol en fonction de l'importance du niveau d'écrasement du talon. Par contre il ne permet pas, comme un pied naturel, d'agir sur la prise d'appui au sol immédiatement après l'attaque du talon.

D'autre part, ce pied prothétique met en oeuvre de nombreux éléments en mouvement, en glissement et en frottement, ce qui, outre le coût élevé de fabrication, nécessite d'une part une mise au point particulière, et d'autre part un entretien permanent.

On connaît également par le document FR 2 839 443 un pied artificiel autopropulsif à énergie embarquée. Cette chaussure comprend deux sous-ensembles qui se croisent et sont articulés l'un à l'autre au travers d'un axe transversal, tandis que des actionneurs sont intercalés entre chacune de leurs parties extrêmes, actionneurs qui consistent en des vessies gonflables ou analogues. L'alternance du gonflement et du dégonflement des vessies, provoque une succession de mouvements de ciseaux des sous-ensembles susceptibles de simuler le déroulé du pied.

Ce pied artificiel présente cependant l'inconvénient, outre d'être complexe à mettre en oeuvre, d'être tributaire d'actionneurs utilisant une source d'énergie extérieure.

Il n'existe pas actuellement de pied prothétique permettant une attaque du talon qui, tout en étant d'une certaine souplesse, génère la prise d'appui immédiate de l'avant du pied.

La présente invention a pour but de proposer un pied prothétique permettant de remédier aux divers inconvénients précités.

Le pied prothétique selon l'invention est du type à restitution d'énergie, comprenant au moins une lame ressort en matériau composite solidaire fixement de la cheville ou du tibia, ou d'un support fixé à ladite cheville ou audit tibia, et destinée à permettre une prise d'appui dynamique au sol, et il se caractérise essentiellement en ce qu'il comporte de plus un bras rigide relié à ladite lame au travers d'un premier moyen de liaison qui autorise une articulation en pivotement dudit bras sur ladite lame selon un axe transversal à cette dernière; et en ce que ledit bras rigide comporte une partie destinée à permettre une autre prise d'appui au sol sensiblement à la verticale, de préférence en arrière, de ladite cheville ou dudit tibia; et ce que encore, ledit bras rigide et ladite lame sont reliés par un second moyen de liaison disposé en une zone distante dudit premier moyen de liaison.

Selon un mode de réalisation particulier du pied prothétique selon l'invention, le premier moyen de liaison relie directement le bras à la lame.

Selon un autre mode de réalisation particulier du pied prothétique selon l'invention, le premier moyen de liaison relie le bras au support de la lame.

Selon une caractéristique additionnelle du pied prothétique selon l'invention, le premier moyen de liaison se présente sous la forme d'un moyen d'articulation en pivotement sur un arbre.

Selon une autre caractéristique additionnelle du pied prothétique selon l'invention, le premier moyen de liaison est un moyen élastique.

Selon une autre caractéristique additionnelle du pied prothétique selon l'invention, le second moyen de liaison se présente sous la forme d'un moyen apte à venir prendre appui sur la lame.

Selon une autre caractéristique additionnelle du pied prothétique selon l'invention, le second moyen de liaison se présente sous la forme d'un moyen de liaison élastique.

L'élasticité des moyens de liaison, que ce soit au niveau du premier moyen de liaison et/ou du second, présente en outre l'avantage de permettre un mouvement d'éversion / inversion.

Selon une autre caractéristique additionnelle du pied prothétique selon l'invention, il comporte des moyens aptes à permettre de faire varier la distance qui sépare les deux moyens de liaison.

Par la variation du bras de levier du bras sur la lame, on modifie les efforts exercés sur celle-ci par ledit bras, réglant du même coup la souplesse du talon et la durée du contact du pied au sol.

Selon une autre caractéristique additionnelle du pied prothétique selon l'invention, il comporte des moyens aptes à permettre de régler la distance qui sépare le support de la lame par rapport à la partie du bras destinée à prendre appui au sol.

Quel que soit le mode de réalisation, le pied prothétique selon l'invention présente de nombreux avantages par rapport à ceux existants, notamment en permettant une mise à plat pus rapide et plus longue du pied, générant une meilleure stabilité du patient, surtout lors des passages en déclivité, et une transition continue sur une même lame travaillant dans les deux sens lors du passage du pas.

Les avantages et les caractéristiques du pied prothétique selon l'invention, ressortiront plus clairement de la description qui suit et qui se rapporte au dessin annexé, lequel en représente plusieurs modes de réalisation non limitatifs.

Dans le dessin annexé :
- la figure 1 représente une vue schématique de profil d'un premier mode de réalisation du pied prothétique selon l'invention.
- la figure 2 représente une vue schématique de dessus du même pied prothétique.
- les figures 3a, 3b, 3c et 3d représentent des vues schématiques de profil du même pied prothétique lors de phases successives de la marche.
- les figures 4 et 5 représentent des vues schématiques en perspective de variantes du pied prothétique selon l'invention.
- la figure 6 représente une vue schématique de profil d'une autre variante du même du pied prothétique selon l'invention.
- la figure 7 représente une vue schématique de profil d'un autre mode de réalisation du même pied prothétique selon l'invention.

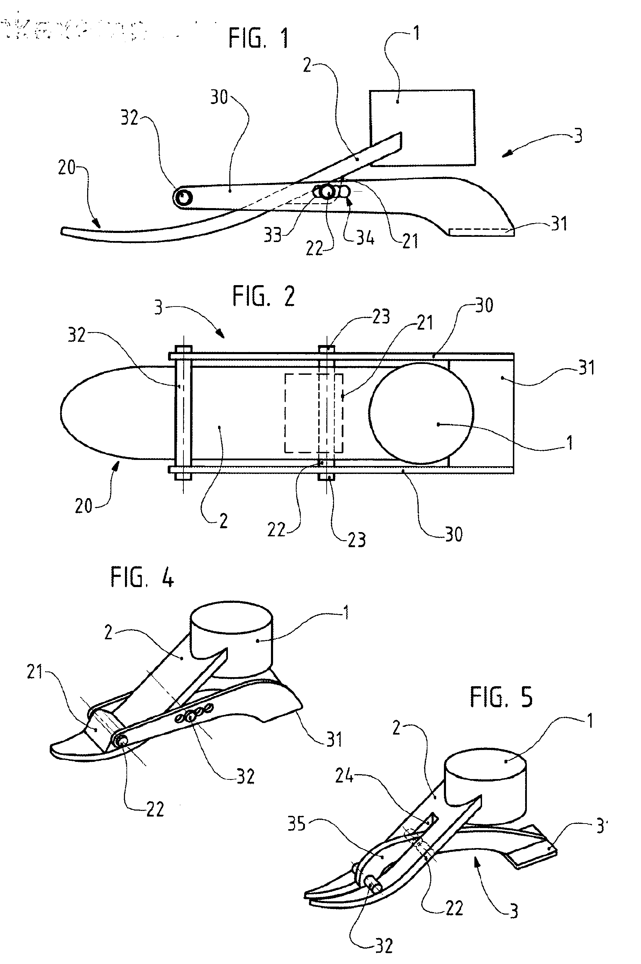

En référence aux figures 1 et 2, on peut voir un pied prothétique selon l'invention, lequel comprend une base 1 destinée à être fixée à l'extrémité d'une jambe, et sur laquelle est solidarisée fixement une lame ressort 2 réalisée en matériau composite, de la fibre de verre par exemple. La lame ressort 2 s'étend vers l'avant du pied et selon un certain angle, en sorte que sa partie distale 20 puisse permettre une prise d'appui dynamique au sol.

On notera que différentes configurations sont réalisables au niveau du montage du pied prothétique, il peut par exemple être prévu d'intercaler entre celui-ci et l'emboîture du tibia, des moyens de liaison soit rigides, soit souples.

La lame ressort 2 présente inférieurement dans sa région médiane une chape 21 traversée par un arbre transversal 22, lequel consiste en un premier moyen de liaison à un bras rigide 3, liaison réalisée de manière pivotante.

Le bras 3 comprend deux flasques latéraux 30, montés chacun sur une extrémité 23 de l'arbre 22, et reliés à l'arrière du pied par une traverse 31 et à l'avant par un arbre transversal 32 qui passe au-dessus de la lame ressort 2, et qui constitue un second moyen de liaison.

La traverse 31 est prévue apte à constituer un appui, et plus particulièrement elle forme le talon du pied prothétique.

L'arbre 22 traverse chacun des flasques 30 par l'un des trous 33 d'une série 34 de trous répartis longitudinalement, en sorte qu'il est possible de choisir de positionner l'arbre 22 plus ou moins loin de l'arbre 32.

Le bras 3 est mobile en pivotement par rapport à la lame ressort 2, ce pivotement est libre, mais toutefois limité dans le sens de l'extension du pied, par l'arbre transversal 32 qui vient buter contre la lame ressort 2.

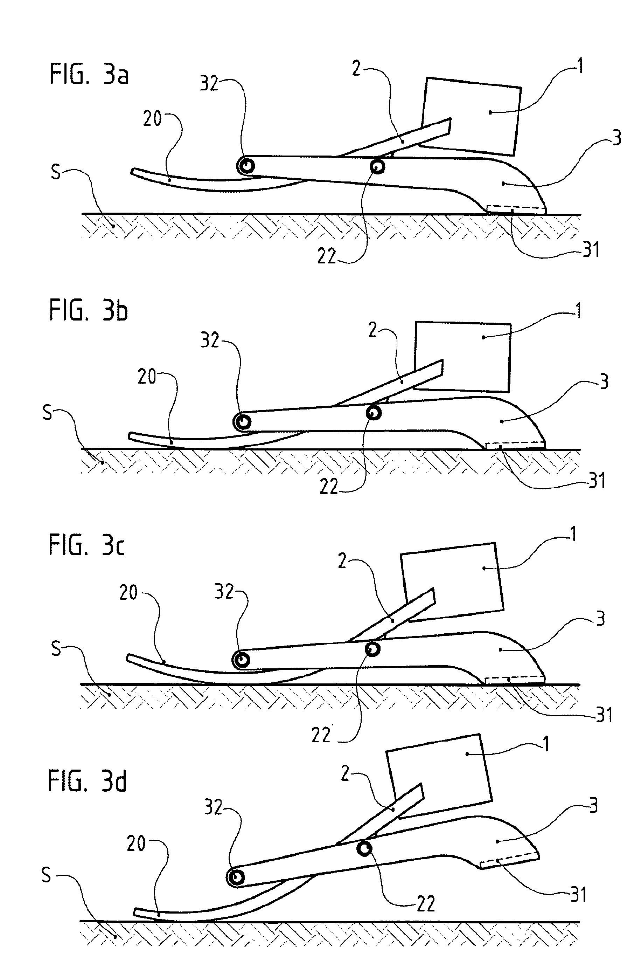

En référence maintenant aux figures 3a, 3b, 3c et 3d on peut voir le comportement du même pied prothétique lors la pratique de la marche.

Sur la figure 3a, le pied prothétique attaque le sol S par le talon, c'est-à-dire par la traverse 31. La force exercée sur la base 1 provoque la remontée de la lame ressort 2 en butée contre l'arbre transversal 32, en sorte que la base 1 se trouve suspendue à l'extrémité de la lame ressort 2, la pose du talon se trouve donc amortie, tandis que cela provoque le placage de la lame ressort 2 contre le sol S.

Sur la figure 3b, le pied prothétique est en appui stable sur le sol S, à la fois par l'intermédiaire de la lame ressort 22 et par la traverse 31, la lame ressort 2 étant de plus arcboutée contre l'arbre 32, en sorte que la base 1 est suspendue.

Sur la figure 3c, le poids du patient passe vers l'avant, mais la lame 2 et le talon 31 demeurent au sol, procurant ainsi une plus grande stabilité.

Sur la figure 3d, le pied passe en appui sur l'avant du pied, donc uniquement sur la partie distale 20 de la lame ressort 2, le bras 3 n'a donc aucune action.

On notera à ce sujet qu'il peut être prévu des moyens de rappel limitant l'ouverture angulaire entre la lame ressort 2 et le bras 3, et plus exactement l'écart entre la base 1 et la traverse 31. Le pied prothétique est cependant destiné à être contenu dans une enveloppe esthétique, laquelle peut avoir cette capacité de limiter l'ouverture angulaire.

Le pied prothétique selon l'invention présente ainsi un déroulé très proche de celui d'un pied naturel. A l'attaque du talon il y a un léger écrasement de celui-ci, écrasement qui reste dynamique, et qui génère en réaction une prise de contact rapide de la lame ressort 2 avec le sol, effet qui n'est possible avec les pieds prothétiques existants, tels que celui du document US 6 767 370.

En référence maintenant aux figures 4, 5 et 6, on peut voir des variantes du pied prothétique selon l'invention.

A la figure 4 est représenté un pied prothétique pratiquement identique à celui représenté sur les figures 1 à 3, et qui n'en diffère que du fait de l'inversion des moyens de liaison, à savoir de l'arbre de pivotement 22 et de l'arbre transversal d'appui 32, en sorte que l'articulation du bras 3 est réalisée en extrémité de celui-ci, à l'opposé de la traverse 31. Ce pied prothétique permet le même déroulé que celui représenté sur les figures 1 à 3.

A la figure 5 est représenté un pied prothétique également pratiquement identique à celui représenté sur les figures 1 à 3, où les arbres de pivotement 22 et d'appui 32 ne sont pas inversés, mais qui diffère en ce que le bras rigide 3 ne comporte pas deux flasques latéraux, mais un seul corps central 35 qui passe dans une fente longitudinale 24 que comporte la lame ressort 2.

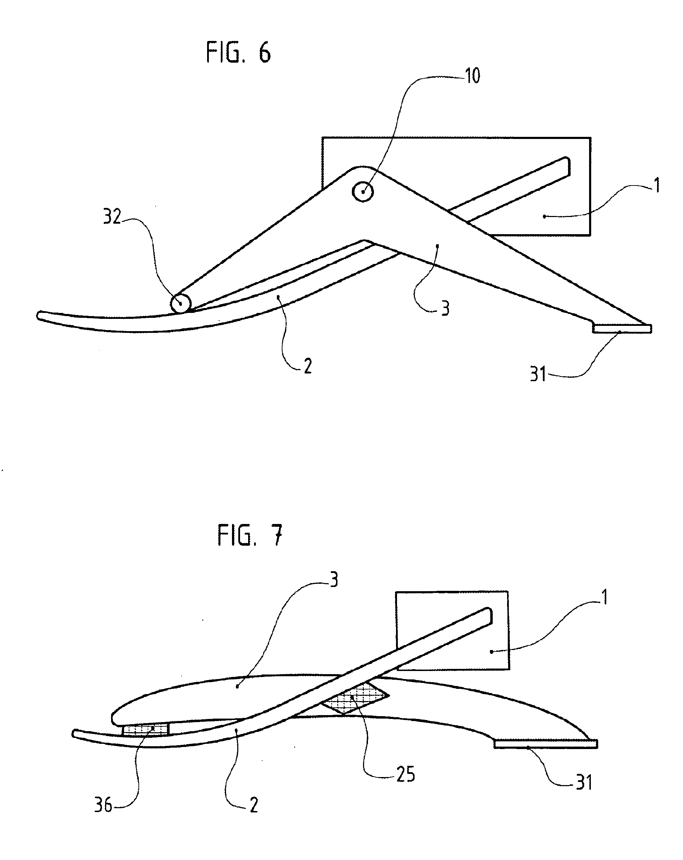

A la figure 6 est représentée une autre variante du pied prothétique selon l'invention, où le bras rigide 3 est monté pivotant, non pas directement sur la lame 2, mais sur la base 1, par l'intermédiaire d'un arbre 10.

On notera que, quel que soit le mode de réalisation, il peut être prévu, outre le réglage de la distance séparant les arbres de pivotement 22, 10 et d'appui 32, des moyens permettant de régler la distance séparant la base 1 de la traverse 31 ou analogue, c'est-à-dire de régler la hauteur du talon prothétique. Ce réglage peut être réalisé de différentes manières, telles que, non limitativement, soit une chape 21 mobile en déplacement longitudinal le long de la lame 2, soit une chape 21 présentant une série de plusieurs trous pour le passage de l'arbre 22, et s'étendant non parallèlement au plan général de la lame 2, soit une série de trous dans la base 1 pour le passage de l'arbre 10, soit une série de trous 33 s'étendant non parallèlement à l'axe principal du bras 3.

En référence maintenant à la figure 7, on peut voir un autre mode de réalisation du pied prothétique selon l'invention, dans lequel le bras 3 est lié à la lame 2 par l'intermédiaire d'une part d'un premier moyen de liaison 25 qui consiste en une liaison élastique, qui autorise un mouvement de pivotement du bras 3 sur la lame 2 ; et par d'autre part un second moyen de liaison 36 disposé entre la lame 2 et l'extrémité du bras 3 opposée à la partie d'appui 31, ce second moyen de liaison étant également une liaison élastique.

Les moyens de liaison 25 et 36 peuvent consister en des éléments en matière plastique, caoutchouc ou autres matériaux présentant des propriétés analogues, solidarisés, par collage par exemple, entre la lame 2 et le bras rigide 3, ou une partie de celui-ci.

Ces moyens de liaison 25 et 36 pourront également être assujettis à la lame 2 et ou au bras rigide 3 au travers d'un moyen de solidarisation amovible et mobile, afin de pouvoir modifier la distance qui les sépare.

Un tel mode de réalisation, permet de lier plus étroitement la lame 2 et le bras rigide 3, et autorise une certaine souplesse dans le sens transversal, et permet un mouvement d'éversion / inversion.

## Revendications

1. Pied prothétique, du type à restitution d'énergie, comprenant au moins une lame ressort (2) en matériau composite solidaire fixement de la cheville ou du tibia, ou d'un support (1) fixé à ladite cheville ou audit tibia, et destinée à permettre une prise d'appui dynamique au sol (S), **caractérisé en ce qu'**il comporte de plus un bras rigide (3) relié à ladite lame (2) au travers d'un premier moyen de liaison (10 ; 22 ; 25) qui autorise une articulation en pivotement dudit bras (3) sur ladite lame (2) selon un axe transversal à cette dernière; et **en ce que** ledit bras rigide (3) comporte une partie (31) destinée à permettre une autre prise d'appui au sol sensiblement à la verticale, de préférence en arrière, de ladite cheville ou dudit tibia; et **en ce que** encore, ledit bras rigide (3) et ladite lame (2) sont reliés par un second moyen de liaison (32 ; 36) disposé en une zone distante dudit premier moyen de liaison (10 ; 22 ; 25).

2. Pied prothétique selon la revendication 1, **caractérisé en ce que** le premier moyen de liaison (22 ; 25) relie directement le bras (3) à la lame (2).

3. Pied prothétique selon la revendication 1, **caractérisé en ce que** le premier moyen de liaison (10) relie le bras (3) au support (1) de la lame (2).

4. Pied prothétique selon l'une quelconque des revendications précédentes, **caractérisé en ce que** le premier moyen de liaison (22 ; 25) se présente sous la forme d'un moyen d'articulation en pivotement sur un arbre.

5. Pied prothétique selon l'une quelconque des revendications 1 à 3, **caractérisé en ce que** le premier moyen de liaison (10) est un moyen de liaison élastique.

6. Pied prothétique selon l'une quelconque des revendications précédentes, **caractérisé en ce que** le second moyen de liaison (32) se présente sous la forme d'un moyen apte à venir prendre appui sur la lame (2).

7. Pied prothétique selon l'une quelconque des revendications 1 à 5, **caractérisé en ce que** le second moyen de liaison (36) se présente sous la forme d'un moyen de liaison élastique.

8. Pied prothétique selon l'une quelconque des revendications précédentes, **caractérisé en ce qu'**il comporte des moyens (33, 34) aptes à permettre de faire varier la distance qui sépare les deux moyens de liaison (10, 22, 32; 25, 36).

9. Pied prothétique selon l'une quelconque des revendications précédentes, **caractérisé en ce qu'**il comporte des moyens aptes à permettre de régler la distance qui sépare le support (1) de la lame (2) par rapport à la partie (31) du bras (3) destinée à prendre appui au sol (S).

## Claims

1. Prosthetic foot of the type with energy restitution, comprising at least one blade spring (2) made of composite material integral with the ankle or the tibia or a support (1) secured to said ankle or said tibia, and aimed at permitting a dynamic rest on the ground (S), wherein it includes addiction a rigid arm (3) connected to said blade (2) through a first connecting means (10; 22; 25), which permit a pivoting hinging of said arm (3) on said blade (2) according to an axis transversal to the latter, and wherein said rigid arm (3) includes a portion (31) aimed at permitting another, substantially vertical, preferable rearward rest on the ground, of said ankle or said tibia; and wherein also said rigid arm (3) and said blade (2) are connected by a second connecting means (32; 36) arranged in an area remote from said first connecting mean (10; 22; 25).

2. Prosthetic foot according to claim 1, wherein the first connecting means (22; 25) connects the arm (3) directly to the blade (2).

3. Prosthetic foot according to claim 1, wherein the first connecting means (10) connects the arm (3) to the support (1) of the blade (2).

4. Prosthetic foot according to any of the preceding claims, wherein the first connecting means (22; 25) is in the form of a means for pivotally hinging to a shaft.

5. Prosthetic foot according to any of claims 1 to 3, wherein the first connecting means (10) is an elastic connecting means.

6. Prosthetic foot according to any of the preceding claims, wherein the second connecting means (32) is in the form of a means capable of resting on the blade (2).

7. Prosthetic foot according to any of claims 1 to 5, wherein the second connecting means (36) is in the form of is an elastic connecting means.

8. Prosthetic foot according to many of the preceding claims, wherein it includes means (33, 34) capable of permitting to cause the distance separating the two connecting means (10, 22, 32; 25, 36) to vary.

9. Prosthetic foot to any of the preceding claim, wherein it includes means capable of permitting to adjust the distance separating the support (1) of the blade (2) from the portion (31) of the arm aimed at resting on the ground (S).

## Patentansprüche

1. Prothetischer Fuß der Art mit Energierückgabe, umfassend zumindest eine Blattfeder (2) aus Verbundwerkstoff, die mit dem Fußknöchel oder mit dem Schienbein oder mit einem Träger (1) fest verbunden ist, der an dem besagten Fußknöchel oder an dem besagten Schienbein befestigt ist, und die vorgesehen ist, um eine dynamische Abstützung auf dem Boden (S) zu erlauben, **dadurch gekennzeichnet, dass** er außerdem ein starrer Arm (3) umfasst, der mit der besagten Blattfeder (2) über ein erstes Verbindungsmittel (10; 22; 25) verbunden ist, das eine gelenkige Verbindung in Schwenkung des besagten Armes (3) mit der besagten Blattfeder (2) nach einer Achse erlaubt, die zu dieser letzteren quer verlauft, und dadurch, dass der besagte starre Arm (3) einen Teil (31) umfasst, der vorgesehen ist, um eine weitere, im Wesentlichen vertikale Abstützung auf dem Boden, vorzugsweise rückwärts, des besagten Fußknöchels oder des besagten Schienbeins zu erlauben; und auch noch dadurch, dass der besagte starre Arm (3) und die besagte Blattfeder (2) durch ein zweites Verbindungsmittel (32; 36) verbunden sind, das in einem von dem ersten Verbindungsmittel (10; 22; 25) entfernten Bereich ungeordnet ist.

2. Prothetischer Fuß nach Anspruch 1, **dadurch gekennzeichnet, dass** das erste Verbindungsmittel (22; 25) den Arm (3) unmittelbar mit der Blattfeder (2) verbindet.

3. Prothetischer Fuß nach Anspruch 1, **dadurch gekennzeichnet, dass** das erste Verbindungsmittel (10) den Arm (3) mit dem Träger (1) der Blattfeder (2) verbindet.

4. Prothetischer Fuß nach irgendeinem der vorgehenden Ansprüche, **dadurch gekennzeichnet, dass** das erste Verbindungsmittel (22; 25) als ein Mittel für die gelenkige Verbindung in Schwenkung auf einem Schaft ausgestaltet ist.

5. Prothetischer Fuß nach irgendeinem der Ansprüche 1 bis 3, **dadurch gekennzeichnet, dass** das erste Verbindungsmittel (10) ein elastisches Verbindungsmittel ist.

6. Prothetischer Fuß nach irgendeinem der vorgehenden Ansprüche, **dadurch, gekennzeichnet, dass** das zweite Verbindungsmittel (32) als ein Mittel ausgestaltet ist, das geeignet ist, um auf der Blattfeder (2) zum Aufliegen zu kommen.

7. Prothetischer Fuß nach irgendeinem der Anspruche 1 bis 5, **dadurch gekennzeichnet, dass** das zweite Verbindungsmittel (36) als ein elastisches Verbindungsmittel ausgestaltet ist.

8. Prothetischer Fuß nach irgendeinem der vorgehenden Ansprüche, **dadurch gekennzeichnet, dass** er Mittel (33, 34) umfasst, die geeignet sind, um zu erlauben, die Entfernung variieren zu lassen, die die beiden Verbindungsmittel (10, 22, 32; 25, 36) trennt.

9. Prothetischer Fuß nach irgendeinem der vorgehenden Ansprüche, **dadurch gekennzeichnet, dass** er Mittel umfasst, die geeignet sind, um zu erlauben, die Entfernung einzustellen, die den Träger (1) der Blattfeder (2) von dem Teil (31) des Armes, vorgesehen, um auf dem Boden (S) abzustützen, trennt.
